Europäisches Patentamt

European Patent Office

Office européen des brevets

(11) Numéro de publication: **0 371 884**
**A1**

(12) # DEMANDE DE BREVET EUROPEEN

(21) Numéro de dépôt: **89420402.3**

(51) Int. Cl.5: **C07C 205/06, C07C 201/08**

(22) Date de dépôt: **20.10.89**

(30) Priorité: **24.10.88 FR 8814228**

(43) Date de publication de la demande:
**06.06.90 Bulletin 90/23**

(84) Etats contractants désignés:
**BE DE ES FR GB IT NL**

(71) Demandeur: **RHONE-POULENC CHIMIE**
**25, quai Paul Doumer**
**F-92408 Courbevoie Cédex(FR)**

(72) Inventeur: **Dcussain, Claude**
**2, rue Louis Girardet**
**F-69190 Saint-Fons(FR)**
Inventeur: **Gubelmann, Michel**
**39, Boulevard des Belges**
**F-69006 Lyon(FR)**
Inventeur: **Popa, Jean-Michel**
**2, rue Roger Breton**
**F-93700 Drancy(FR)**

(74) Mandataire: **Varnière-Grange, Monique et al**
**RHONE-POULENC CHIMIE Service Brevets**
**Chimie Centre de Recherches des Carrières**
**B.P. 62**
**F-69192 Saint-Fons Cédex(FR)**

(54) **Procédé de préparation de dinitrotoluènes.**

(57) La présente invention concerne un procédé de préparation du dinitrotoluène par réaction du mononitrotoluène et de l'acide nitrique en phase liquide. Le procédé est caractérisé en ce que la réaction est conduite en présence d'un solide minéral et acide comprenant au moins un composant choisi dans le groupe constitué par l'acide orthophosphorique, l'acide pyrophosphorique, l'acide métaphosphorique, les dihydrogénophosphates métalliques, les hydrogénophosphates métalliques, les orthophosphates métalliques et les pyrophosphates métalliques dérivant des métaux susceptibles de former des cations porteurs de 1 à 5 charges positives.

EP 0 371 884 A1

## PROCEDE DE PREPARATION DE DINITROTOLUENES

La présente invention concerne un procédé de préparation de dinitrotoluènes. Elle a plus particulièrement trait à un procédé de préparation de dinitrotoluènes par réaction d'au moins un mononitroluène et de l'acide nitrique en phase liquide en présence d'un solide acide.

Les dinitrotoluènes, en particulier le dinitro-2,4 toluène, le cas échéant en mélange avec le dinitro-2,6 toluène, sont des produits intermédiaires de la chaîne de fabrication du toluène-diisocyanate (TDI) qui entre dans la préparation de divers polyuréthanes. Le dinitro-2,4 toluène est donc produit industriellement et à un très grande échelle. Dans un tel contexte il est aisé de concevoir l'importance que peut revêtir une augmentation de quelques pourcents sur un taux de conversion et/ou sur une sélectivité, par exemple, ou une amélioration au niveau de l'une quelconque des étapes de mise en oeuvre du procédé.

Le dinitro-2,4 toluène est classiquement produit par une double nitration en phase liquide du toluène au moyen d'un mélange d'acide nitrique et d'acide sulfurique concentré. La concentration de l'acide sulfurique utilisé pour la deuxième nitration (nitration du mononitrotoluène en dinitrotoluène) étant typiquement de l'ordre de 98 %. Nonobstant les problèmes de corrosion et de coûts importants du recyclage de grands volumes d'acide sulfurique, le procédé présente des inconvénients importants que sont le caractère problématique de l'élimination du catalyseur, résolu généralement par des lavages à l'eau, et la nécessité de concentrer l'acide sulfurique avant son recyclage dans la mesure où la réaction elle-même génère de l'eau.

Il serait donc souhaitable de pouvoir disposer d'un procédé efficace de préparation du dinitrotoluène qui permette d'obvier au moins partiellement aux inconvénients précités.

La présente invention a donc pour objet un procédé de préparation de dinitrotoluènes par réaction d'au moins un mononitrotoluène et de l'acide nitrique en phase liquide caractérisé en ce que la réaction est conduite en présence d'un solide minéral et acide comprenant au moins un composant choisi dans le groupe constitué par l'acide orthophosphorique, l'acide pyrophosphorique, l'acide métaphosphorique, les dihydrogénophosphates métalliques, les hydrogénophosphates métalliques, les orthophosphates métalliques et les pyrophosphates métalliques dérivant des métaux susceptibles de former des cations porteurs de 1 à 5 charges positives.

Par dinitrotoluène(s) on entend, de préférence, dans le cadre du présent procédé le dinitro-2,4 toluène, le dinitro-2,6 toluène et leurs mélanges.

Par nitrotoluène(s) on entend le p-nitrotoluène, l'o-nitrotoluène et leurs mélanges.

Bien entendu, si la matière de départ est un mélange d'ortho- et de para-nitrotoluène on obtiendra un mélange d'isomères 2,4 et 2,6 du dinitrotoluène ; si la matière de départ est le para-nitrotoluène, le dinitro-2,4 toluène sera le seul produit observé.

Le procédé selon l'invention requiert la présence d'acide nitrique. On recourt généralement à des formes concentrées d'acide nitrique, la concentration étant supérieure à 90 % et de préférence, comprise entre 95 et 100 %.

L'une des caractéristiques essentielles du présent procédé réside dans l'utilisation d'un solide minéral et acide.

Par solide on entend un composé ou une composition pratiquement insoluble dans les réactifs et le produit organique visé, et le cas échéant, pratiquement insoluble dans un diluant de nature organique et de préférence, également pratiquement insoluble dans l'eau.

Un tel composé (ou composition) de nature essentiellement minérale pourra se présenter sous des formes massiques ou dispersées telles que billes, pastilles, extrudés et poudres dont les dimensions moyennes résulteront généralement d'un compromis entre l'activité recherchée, le coût et le choix des techniques de séparation choisies.

Par acidité on entend l'acidité de Lewis et/ou de Bronsted.

L'acidité de Bronsted peut être intrinsèque au composé (ou à la composition) choisi(e) ou créée par la présence d'eau.

Une autre caractéristique essentielle du présent procédé réside dans le fait que le solide comprend au moins un composant choisi dans le groupe constitué par l'acide orthophosphorique, l'acide pyrophosphorique, l'acide métaphosphorique, les dihydrogénophosphates métalliques, les hydrogénophosphates métalliques, les orthophosphates métalliques et les pyrophosphates métalliques dérivant des métaux susceptibles de former des cations porteurs de 1 à 5 charges positives.

Par phosphates on entend les sels d'un métal susceptible de former des cations porteurs de 1 à 5 charges positives de l'acide orthophosphorique dans lequel au moins un atome d'hydrogène a été remplacé par un atome métallique.

Par pyrophosphates on entend les sels d'un métal susceptible de former des cations porteurs de 1 à 5 charges positives de l'acide pyrophosphorique dans lequel au moins un atome d'hydrogène a été remplacé par un atome métallique.

A titre d'exemples d'orthophosphates métalliques susceptibles d'entrer dans la composition du solide utilisé selon l'invention on peut citer :

$Li_3PO_4$ , $Ca_3(PO_4)_2$ , $Mg_3(PO_4)_2$ , $Cu_3(PO_4)_2$ , $Zn_3(PO_4)_2$ , $Mn_3(PO_4)_2$ , $Co_3(PO_4)_2$ , $Ni_3(PO_4)_2$ , $BPO_4$ , $CrPO_4$ , $FePO_4$ , $LaPO_4$ , $AlPO_4$ , $CePO_4$ , $Ti_3(PO_4)_4$ , $Ce_3(PO_4)_4$, $Si_3(PO_4)_4$ , $Nb_3(PO_4)_5$

A titre d'exemples de monohydrogénophosphates métalliques susceptibles d'entrer dans la composition du solide utilisé dans le cadre de la présente invention on peut citer :

$CaHPO_4$ , $Ti(HPO_4)_2$ , $Zr(HPO_4)_2$ , $MgHPO_4$

A titre d'exemples de pyrophosphates métalliques susceptibles d'entrer dans la composition du solide utilisé dans le cadre de la présente invention on peut citer :

$Ca_2P_2O_7$ , $Cu_2P_2O_7$ , $Fe_4(P_2O_7)_3$ , $TiP_2O_7$

Parmi les divers types de composés métalliques énoncés précédemment, on recourt avantageusement dans le cadre du procédé selon l'invention à ceux dérivant des métaux susceptibles de former des cations porteurs d'au moins deux charges positives et, de préférence, de 3 à 5 charges positives.

Pour une bonne mise en oeuvre de l'invention on recourra, de préférence, à un catalyseur comprenant au moins un composant choisi dans le groupe constitué par l'acide orthophosphorique, l'acide pyrophosphorique, l'orthophosphate de silicium, l'orthophosphate de lanthane et leurs mélanges. Comme indiqué en tête du présent mémoire le solide peut se présenter sous forme massique ou dispersée. Il peut également se présenter sous forme d'un support solide sur lequel est attaché ou dispersé au moins l'un des composants mentionnés précédemment. Le support peut être choisi parmi les oxydes classiquement utilisés comme supports de catalyseurs tels les silices, les alumines, les oxydes de titane, les oxydes de zirconium et les oxydes de terres rares.

Certains composants entrant dans la constitution du solide utilisé dans le cadre de la présente invention sont des produits courants du commerce ; d'autres composants sont connus en eux-mêmes et peuvent être préparés par des méthodes appropriées décrites dans la littérature.

La quantité de solide à mettre en oeuvre dans le cadre du présent procédé n'est pas critique et dépendra généralement d'un compromis entre l'activité recherchée, le coût du solide et les diverses autres conditions de mise en oeuvre du procédé.

En général la quantité de solide sera telle que le rapport pondéral de l'acide nitrique au solide soit compris entre 0,02 et 10. Pour une bonne mise en oeuvre du procédé selon l'invention ce rapport sera compris entre 0,1 et 2.

La réaction entre le(s) mononitrotoluène(s) et l'acide nitrique est conduite en phase liquide, le rapport molaire de l'acide nitrique au(x) mononitrotoluène(s) pouvant varier dans de larges limites. Pour une bonne mise en oeuvre le rapport molaire sera compris entre 0,1 et 10.

En général, une température d'au moins 40°C s'avère nécessaire pour obtenir une vitesse de conversion acceptable et au-delà de 100°C, des réactions parasites sont susceptibles de se produire et de diminuer l'intérêt d'un tel procédé.

Selon une variante avantageuse du présent procédé le milieu réactionnel liquide renfermera également un diluant choisi parmi les composés organiques inertes vis-à-vis de la réaction de nitration et stables dans les conditions de mise en oeuvre de la réaction. De préférence, le diluant liquide sera en outre de nature telle que l'acide nitrique et le(s) mononitrotoluène(s) soient le moins possible solubles dans le diluant.

A titre d'exemples de tels diluants on peut citer :

- les hydrocarbures halogénés, saturés acycliques et en particulier le chlorure de méthylène et le dichloro-1,2 éthane,
- les dérivés des hydrocarbures renfermant du chlore et du fluor plus communément désignés par Fréon[R] et en particulier le F-113,
- les (poly)cycloalcanes perfluorés comportant de 5 à 12 atomes de carbones, notamment le composé dérivant du cyclohexane ou du méthyl-cyclohexane par remplacement de tous les atomes d'hydrogène par des atomes de fluor et la perfluorodécaline,
- les mononitroalcanes comportant de 1 à 4 atomes de carbone et
- le m-nitrochlorobenzène.

On recourt avantageusement à la perfluorodécaline ou au chlorure de méthylène.

Pour une bonne mise en oeuvre de cette variante, la Demanderesse préconise l'utilisation de 0,5 à 5 ml de diluant par g de solide engagé, des rapports différents pouvant être utilisés en pratique pour des raisons économiques et/ou des raisons liées à la conception même du réacteur choisi pour assurer un bon contact liquide-solide.

La réaction est généralement conduite dans un dispositif assurant un bon contact liquide-solide tel un réacteur agité ou un réacteur avec boucle de circulation de liquide.

Au bout du temps fixé pour la réaction (ou du temps de séjour souhaité) on récupère le(s) dinitrotoluène(s) par tout moyen approprié, par exemple par distillation le cas échéant précédée par la décantation ou la filtration du catalyseur.

Les exemples ci-après illustrent l'invention.

EXEMPLES 1 à 5 : Essai Témoin a :

Dans un ballon tricol de 50 cm3 muni d'une agitation centrale et d'un réfrigérant on introduit à 25° C 4 g de solide dont la nature, la provenance commerciale et, le cas échéant, les conditions de traitement préalable sont précisées dans le tableau ci-après, 8 cm3 de perfluorodécaline (PFD ; $C_{10}F_{18}$), 2,1 g (15 mmol) de para-nitrotoluène, 1 g (16,1 mmol) d'acide nitrique à 100 %. Le mélange est porté à 60° C pendant 4 heures sous agitation. Le mélange résultant est filtré ; le filtrat est lavé avec de l'eau.

La phase organique est alors analysée par chromatographie en phase gazeuse.

Les conditions particulières ainsi que les résultats obtenus figurent dans le tableau ci-après dans lequel TT désigne le taux de transformation du p-nitrotoluène
RT désigne le rendement en dinitro-2,4 toluène (DNT).

### TABLEAU

| Réf | Catalyseur | | | TT (%) | RT (%) |
|---|---|---|---|---|---|
| | Nature | Provenance | Traitement | | |
| (a) | Néant | - | - | 27 | 76 |
| 1 | $Nb_3(PO_4)_5$ | CBMM | + | 34 | 90 |
| 2 | $CrPO_4$ | AV | + | 33 | 80 |
| 3 | $H_4P_2O_7$ | FLUKA | Néant | 64.4 | 100 |
| 4 | $LaPO_4, 0.9H_2O$ | AV | + | 58,6 | 88 |
| 5 | $H_3PO_4$ | FLUKA | Néant | 42 | 91 |
| NB : CBMM : société brésilienne | | | | | |
| AV : société Alfa Ventron | | | | | |
| + : traitement thermique à 400° C sous air pendant 3 h | | | | | |

EXEMPLE 6 :

Dans un ballon tricol de 100 cm3 muni d'une agitation centrale et d'un réfrigérant (équipé d'un compte-bulle), on introduit à 25° C 7,63 g de silice phosphatée commerciale (plus connue sous sa désignation commerciale de "Catalyseur UOP") préalablement séchée pendant 4 heures à 220° C sous air et de composition $SiO_2/P_2O_5/H_2O$ = 26/61/13, 6 cm3 de perfluorodécaline (PFD) ($C_{10}F_{18}$) ; 2,74 g (20mM) de para-nitrotoluène (PNT) et 1,29 g (0,84 cm3, 21,4 mM) d'acide nitrique H 100 %. Le mélange est chauffé à 60° C pendant 3,5 heures avec une bonne agitation. La silice phosphatée est récupérée par filtration sur verre fritté et lavée plusieurs fois avec du chlorure de méthylène. Le filtrat est lavé avec de l'eau. La phase organique est analysée par chromatographie en phase gazeuse. On observe une conversion (TT) du PNT de 60,6 % et une sélectivité (RT) en 2,4-DNT de 96,3 %. (La nature du 2,4-DNT a été confirmée par un couplage CPG-spectrométrie de masse).

Essai Témoin b :

On procède comme à l'exemple 6, mais avec des charges de moitié, dans un réacteur cylindrique muni d'une agitation magnétique et en l'absence de solide et de PFD. Les résultats obtenus sont les suivants : TT (PNT) = 37 % RT (2,4-DNT) = 43 %

EXEMPLE 7 :

On procède comme à l'exemple 6, mais avec des charges de moitié, dans un réacteur cylindrique muni d'une agitation magnétique et pendant 30 minutes seulement. Les résultats obtenus sont les suivants : TT (PNT) = 30 % RT (2,4-DNT) = 74 %

EXEMPLE 8 :

On procède comme à l'exemple 7 mais pendant 1,5 heure. Les résultats obtenus sont les suivants : TT (PNT) = 39,6 % RT (2,4-DNT) = 85 %

EXEMPLE 9 :

On procède comme à l'exemple 7 mais pendant 3,5 heures. Les résultats obtenus sont les suivants : TT (PNT) = 47,6 % RT (2,4-DNT) = 89 %

EXEMPLE 10 :

On procède comme à l'exemple 9, mais à 80° C. Les résultats obtenus sont les suivants : TT(PNT) = 60 % RT (2,4-DNT) = 67 %

EXEMPLE 11 :

On procède comme à l'exemple 1, mais avec du FREON[R] 113 (Cl$_2$FCCClF$_2$) comme diluant. Les résultats obtenus sont les suivants : TT(PNT) = 39,9 % RT (2,4-DNT) = 86 %

EXEMPLE 12 :

On procède comme à l'exemple 1, mais avec du PNT comme diluant. Les résultats obtenus sont les suivants : TT(PNT) = 42,2 % RT (2,4-DNT) = 94 %

**Revendications**

1) Procédé de préparation du dinitrotoluène par réaction du mononitrotoluène et de l'acide nitrique en phase liquide caractérisé en ce que la réaction est conduite en présence d'un solide minéral et acide comprenant au moins un composant choisi dans le groupe constitué par l'acide orthophosphorique, l'acide pyrophosphorique, l'acide métaphosphorique, les dihydrogénophosphates métalliques, les hydrogénophosphates métalliques, les orthophosphates métalliques et les pyrophosphates métalliques dérivant des métaux susceptibles de former des cations porteurs de 1 à 5 charges positives.

2) Procédé selon la revendication 1 caractérisé en ce que le solide comprend au moins un composant choisi dans le groupe constitué par l'acide orthophosphorique, l'acide pyrophosphorique, l'acide métaphosphorique, les dihydrogénophosphates métalliques, les hydrogénophosphates métalliques, les orthophosphates métalliques et les pyrophosphates métalliques dérivant des métaux susceptibles de former des cations

5

porteurs d'au moins 2 charges positives.

3) Procédé selon la revendication 1 ou 2, caractérisé en ce que le solide comprend au moins un composant choisi dans le groupe constitué par l'acide orthophosphorique, l'acide pyrophosphorique, l'acide métaphosphorique,les dihydrogénophosphates métalliques, les hydrogénophosphates métalliques, les orthophosphates métalliques et les pyrophosphates métalliques dérivant des métaux susceptibles de former des cations porteurs de 3 à 5 charges positives.

4) Procédé selon l'une quelconque des revendications précédentes caractérisé en ce que le solide comprend au moins un composant choisi dans le groupe constitué par l'acide orthophosphorique, l'acide pyrophosphorique, l'orthophosphate de silicium, l'orthophosphate de lanthane et leurs mélanges.

5) Procédé selon l'une quelconque des revendications précédentes caractérisé en ce que le solide utilisé est le phosphate de lanthane.

6) Procédé selon l'une quelconque des revendications précédentes, caractérisé en ce que la quantité de solide engagée est telle que le rapport pondéral $HNO_3$/solide est compris entre 0,1 et 2.

7) Procédé selon l'une quelconque des revendications précédentes, caractérisé en ce que le rapport molaire $HNO_3$/mononitrotoluène(s) est compris entre 0,1 et 10.

8) Procédé selon l'une quelconque des revendications précédentes, caractérisé en ce que la température de réaction est compris entre 40 et 100°C.

9) Procédé selon l'une quelconque des revendications précédentes, caractérisé en ce que la réaction est conduite en présence d'un diluant.

10) Procédé selon la revendication 9 caractérisé en ce que le diluant est la perfluorodécaline.

11) Procédé selon la revendication 9 caractérisé en ce que le diluant est le chlorure de méthylène.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 202 056 (BASF) <br> * Revendications; page 2, ligne 35 - page 3, ligne 3; exemple 5 * <br> --- | 1-11 | C 07 C 205/06 <br> C 07 C 201/08 |
| A | CH-A- 485 643 (BOFORS) <br> * Exemple 11 * <br> --- | 1-11 | |
| A | US-A-2 400 287 (G.V. CAESAR) <br> * Revendications; page 2, colonne 2, lignes 1-20; exemple x * <br> ----- | 1-11 | |
| | | | **DOMAINES TECHNIQUES RECHERCHES (Int. Cl.5)** |
| | | | C 07 C 201/00 <br> C 07 C 205/00 <br> C 07 B 43/00 |

**Le présent rapport a été établi pour toutes les revendications**

| Lieu de la recherche | Date d'achèvement de la recherche | Examinateur |
|---|---|---|
| LA HAYE | 26-01-1990 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................................................
& : membre de la même famille, document correspondant

EPO FORM 1503 03.82 (P0402)